# EUROPEAN PATENT SPECIFICATION

(11) **EP 2 470 187 B1**
(45) Date of publication and mention of the grant of the patent: **21.08.2013**
(21) Application number: 10747022.1
(22) Date of filing: 23.08.2010
(51) Int. Cl.: A61K 33/44, A61P 39/00, A61P 39/02

(54) **EMERGENCY INTERVENTIONS OF ACTIVE CHARCOAL WITH DABIGATRAN ETEXILATE OVERDOSING**
NOTFALLINTERVENTIONEN MIT AKTIVKOHLE BEI DABIGATRAN ETEXILATE ÜBERDOSIERUNG
INTERVENTIONS D'URGENCE AVEC DU CHARBON ACTIVÉ POUR SURDOSAGE DU DABIGATRAN EXTEXILATE

(30) Priority: 24.08.2009 US 236321 P
(43) Date of publication of application: 04.07.2012
(73) Proprietor: Boehringer Ingelheim International GmbH, 55216 Ingelheim am Rhein (DE)
(72) Inventor: VAN RYN, Joanne, 55216 Ingelheim am Rhein (DE); CLEMENS, Andreas, 55216 Ingelheim am Rhein (DE)
(74) Representative: Simon, Elke Anna Maria
(86) International application number: PCT/EP2010/062229
(87) International publication number: WO 2011/023653

(56) References cited:
- COOPER G M ET AL: "A randomized clinical trial of activated charcoal for the routine management of oral drug overdose" QJM, vol. 98, no. 9, September 2005 (2005-09), pages 655-660, XP002602281 ISSN: 1460-2725
- DATABASE BIOSIS [Online] BIOSCIENCES INFORMATION SERVICE, PHILADELPHIA, PA, US; 2005, KAWASAKI CHIYO I ET AL: "How tightly can a drug be bound to a protein and still be removable by charcoal hemoperfusion in overdose cases?" XP009139148 Database accession no. PREV200510054145 & CLINICAL TOXICOLOGY, vol. 43, no. 2, 2005, pages 95-99, ISSN: 0731-3810(print) 1097-9875(ele
- GUSS D A: "Emergency medicine: activated charcoal-the first-line agent in cases of overdose" WESTERN JOURNAL OF MEDICINE, AFFILIATED CLINICAL INVESTIGATION SOCIETIES, US, vol. 151, no. 1, 1 July 1989 (1989-07-01), page 63, XP009139160 ISSN: 0093-0415
- PRESCOTT L F ET AL: "TREATMENT OF QUININE OVERDOSAGE WITH REPEATED ORAL CHARCOAL" BRITISH JOURNAL OF CLINICAL PHARMACOLOGY, BLACKWELL SCIENTIFIC PUBL, GB, vol. 27, no. 1, 1 January 1989 (1989-01-01), pages 95-97, XP008052949 ISSN: 0306-5251
- DATABASE MEDLINE [Online] US NATIONAL LIBRARY OF MEDICINE (NLM), BETHESDA, MD, US; 1986, POND S M: "A review of the pharmacokinetics and efficacy of emesis, gastric lavage and single and repeated doses of charcoal in overdose patients." XP009139162 Database accession no. NLM2881767 & DEVELOPMENTS IN TOXICOLOGY AND ENVIRONMENTAL SCIENCE 1986 LNKD- PUBMED:2881767, vol. 12, 1986, pages 315-328, ISSN: 0165-2214
- ROTE LISTE FACHINFORMATIONEN: "Drug infomation on Pradaxa®" 1 August 2010 (2010-08-01), XP002602282 Retrieved from the Internet: URL:http://www.fachinfo.de/data/fi/jsearch ?wirkstoff [retrieved on 2010-09-25]

## Description

The invention relates to charcoal for use in the treat an overdosing with active substance dabigatran etexilate of formula I 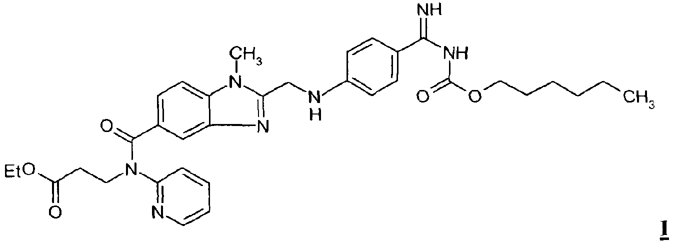 optionally in the form of the pharmaceutically acceptable salts thereof.

### Background to the invention

The compound of formula **1** is known from the prior art and was first disclosed in WO98/37075. It is a potent thrombin inhibitor which can be used for example for the post-operative prevention of deep vein thromboses and in stroke prevention, particularly for preventing strokes in patients with atrial fibrillation. WO 03/074056 discloses the methanesulphonic acid addition salt of dabiagtran-etexilate (ie: dabigatran etexilate methansulphonate) to be particularly useful.

The compound is usually administered orally. In particular, so-called pellet formulations may be used, as disclosed for example in WO 03/074056. These formulations are compositions in which an active substance layer containing binder and optionally separating agent is applied to a substantially spherical core material that consists of or contains a pharmaceutically acceptable organic acid. The core layer and the active substance layer are separated from one another by a so-called isolating layer. The schematic structure of an active substance formulation of this kind is shown in Figure 1 of WO 03/074056.

Dabigatran etexilate (I) is a prodrug, which is rapidly converted in vivo to the active moiety dabigatran (compound of formula II), a potent, direct thrombin inhibitor. 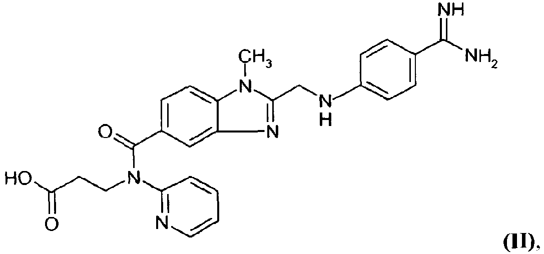 i.e., dabigatran etexilate is only converted into the compound which is actually effective, namely dabigatran, in the body.

This drug is currently being tested for stroke prevention in patients with atrial fibrillation (SPAF) and is administered over longer time periods in these patients as warfarin replacement. Due to risks for bleeding complications under chronic anticoagulant treatment, there may be a need for emergency neutralization of the drug.

It is the objective of the invention in hand to provide for a suitable method for the emergency neutralization of dabigatran etexilate, in particular in case of overdosing.

### Detailed description of the invention

The instant invention is directed to a possible solution of the aforementioned problem. The invention relates to charcoal for use in the treatment of an overdosing with active substance dabigatran etexilate of formula I 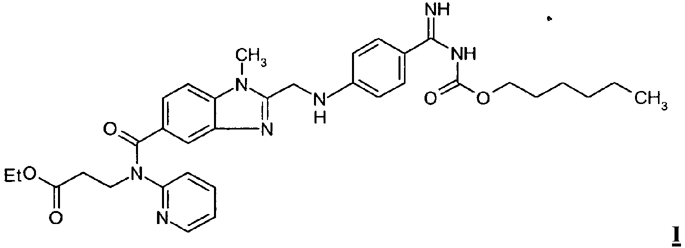 optionally in the form of the pharmaceutically acceptable salts thereof.

The invention relates to the neutralzation of dabigatran etexilate in the stomach of a patient wherein an effective amount of active charcoal is administered orally to a patient in need thereof. The effective amount of active charcoal is usually in a range of 20 - 130 g, preferably in the range of 50 - 100g. The amount of charcoal used depends on the degree of overdosing.

Dabigatran etexilate **I** is a double prodrug of dabigatran, the compound of Formula (**II**) 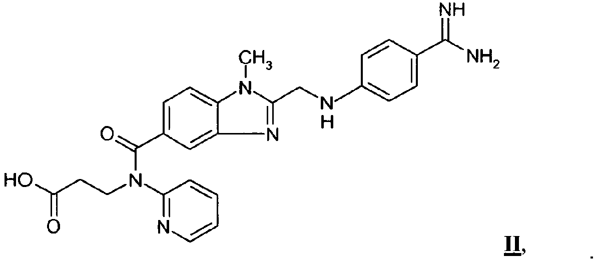 i.e., dabigatran etexilate **I** is only converted into the compound which is actually effective, namely dabigatran **II,** in the body.

In another embodiement the invention relates to the neutralization of dabigatran **II** in the the blood plasma, the method comprising purification of the blood plasma of the patient over active charcoal. The absorption of overdosed dabigatran **II** from the blood plasma could be performed via hemoperfusion over a charcoal filter.

The surprisingly high degree of absorption of dabiagtran etexilate and dabigatran to charcoal was verified in the experiment described herein below. In these experiments, binding of dabigatran etexilate to active charcoal in water simulates recent ingestion (2-5, preferably 2-3 hrs) of large amounts of dabigatran etexilate in the stomach fluid. Binding of dabigatran to active charcoal in plasma simulates situations where dabigatran was absorbed after ingestion and present in high concentrations in plasma.

In the following section a method for the manufacture of dabigatran dosage forms and the dosage form thus obtained are described.

The process for the manufacture of the pharmaceutical compositions used in the mentioned clinical trials is characterised by a series of partial steps. First, the core **1**, is produced from pharmaceutically acceptable organic acid. Within the scope of the present invention tartaric acid is used to prepare the core **1.** The core material **1** thus obtained is then converted into so-called isolated tartaric acid cores **3** by spraying on an isolating suspension **2**. A dabigatran suspension **4** prepared subsequently is sprayed onto these coated cores **3** in one or more process steps by means of a coating process. Finally, the active substance pellets **5** thus obtained are then packed into suitable capsules.

### Determining the particle sizes of tartaric acid by air jet screening

### Measuring device and settings:

| | |
|---|---|
| Measuring device: | Air jet screen, e.g. Alpine A 200 LS |
| Screens: | As required |
| Weight put in: | 10g/screen |
| Duration: | 1 min/screen, then 1 min each up to the maximum weight loss of 0.1g |

### Preparation of sample / supply of product:

The substance is transferred into a mortar and any lumps present are destroyed by intensive pounding. The screen with rubber seal and cover is placed on a balance, set to zero and 10.0 g of the pounded substance are weighed onto the screen.

The screen together with its contents, rubber seal and cover are placed on the device. The timer is set to 1 minute and the material is treated by air jet screening for this time. Then the residue is weighed out and documented. This process is repeated until the decrease in the weight of the residue after air jet screening is < 0.1 g.

### Example 1 - Preparation of the Starter Pellets

480 kg water are heated to 50°C and 120 kg of acacia (gum arabic) are added with stirring in a conventional mixing container having a dished end and stirrer. Stirring is continued at constant temperature until a clear solution is obtained. Once there is a clear solution (usually after 1 to 2 hours) 600 kg tartaric acid are added with stirring. The tartaric acid is added at constant temperature while stirring is continued. After the addition has ended the mixture is stirred for about another 5 to 6 hours.

1000 kg tartaric acid are added to a slowly rotating (3 revolutions per minute) unperforated horizontal pan with a spraying and powder applying unit (e.g. Driamat 2000/2.5). Before spraying starts, a sample of the acid is taken for screening analysis. The acid in question is tartaric acid particles with a particle size in the range from 0.4-0.6 mm. The acid rubber solution obtained by the above method is sprayed onto the tartaric acid particles thus provided. During the spraying, the quantity of air supplied is adjusted to 1000m³/h and 35°-75°C. The differential pressure is 2 mbar and the speed of rotation of the pan is 9 revolutions per minute. The nozzles should be arranged at a distance of 350 - 450 mm from the filling.

The acid rubber solution is sprayed on by alternating with the following steps. After about 4.8 kg of the acid rubber solution has been sprayed onto the tartaric acid particles of particle size 0.4-0.6 mm and the solution has been distributed, about 3.2 kg tartaric acid powder are sprinkled onto the damp tartaric acid particles. The tartaric acid powder in question consists of fine tartaric acid particles with a particle size of < 50 microns. In all, 800 kg tartaric acid powder are required. After the said tartaric acid powder has been sprinkled on and distributed the spray material is dried until a product temperature of about 40°C is reached. This is in turn followed by the spraying on of the acid rubber solution.

These cycles are repeated until the acid rubber solution is used up. Once the process has ended the acid pellets are dried in the pan at 3 rpm for 240 minutes. To prevent caking after the drying has finished, an intermittent program is run at 3 rpm for 3 minutes every hour. In the present instance this means that the pan is rotated at 3 rpm for 3 minutes at intervals of one hour and then left to stand. The acid pellets are then transferred into a dryer. They are then dried at 60°C over a period of 48 hours. Finally, the particle size distribution is determined by screen analysis. The particle size with a diameter of 0.6 - 0.8 mm corresponds to the product. This fraction should make up >85%.

### Example 2 - Isolation of the Starter Pellets

To prepare the isolating suspension, 666.1 (347.5) kg of ethanol are placed in the mixing container and the hydroxypropylmethylcellulose (33.1 (17.3) kg) is added with stirring at approx. 600 rpm and dissolved. Then under the same conditions 0.6 (0.3) kg dimeticone are added. Shortly before use, talc (33.1 (17.3) kg) is added, again with stirring, and suspended.

The acid pellets 1200 (600) kg are poured into the coating apparatus (e.g. GS-Coater Mod. 600/Mod. 1200) and sprayed therein in the rotating pan with the isolating suspension described above in a continuous spraying process lasting several hours at a spraying rate of 32 kg/h for the 1200 kg mixture or 21 kg/h for the 600 kg mixture. The pellets are also dried continuously with an air supply at up to 70°C.

After the GS-Coater has been emptied, the isolated starter pellets are fractionated by screening. The product fraction with a diameter ≤1.0 mm is stored and used further.

### Example 3 - Preparation of the dabigatran etexilate suspension

26.5 kg hydroxypropylcellulose are added to 720 kg isopropanol in a 1200 litre mixing container fitted with a propeller stirrer and the mixture is stirred until fully dissolved (about 12 - 60 hours; roughly 500 rpm). Once the solution is clear, 132.3 kg of dabigatran etexilate methanesulphonate (polymorph I) are added with stirring (400 rpm) and the mixture is stirred for about another 20-30 minutes. Then 21.15 kg of talc is added at a constant stirring rate and stirring is continued at the same speed for about another 10-15 minutes. The steps described above are preferably carried out under a nitrogen atmosphere.

Any clumps formed are broken up by homogenising using an UltraTurrax stirrer (about 60-200 minutes). The suspension temperature should not exceed 30°C throughout the entire manufacturing process.

The suspension is stirred until ready for further processing to ensure that no sedimentation occurs (at roughly 400 rpm).

If the suspension is stored at below 30°C, it should be further processed within at most 48 h. If for example the suspension is manufactured and stored at 22°C, it may be further processed within 60 hours. If the suspension is stored for example at 35°C it should be further processed within at most 24 h.

### Example 4 - Preparation of the dabigatran etexilate active substance pellets

A horizontal pan with an unperforated container is used (GS Coater Mod. 600). In contrast to the fluidised bed method, the suspension is sprayed onto the fluidised bed of pellets in the rotating pan by the "top spray" method. It is sprayed on through nozzles 1.4 mm in diameter. The dry air is passed into the bed of pellets through so-called immersion blades and transported away through an opening in the back wall of the coater.

The horizontal pan is charged with 320 kg of the tartaric acid pellets obtained according to Example 2 and the bed of pellets is heated up. Once a product temperature of 43 °C has been reached, spraying begins. 900 kg of the suspension prepared previously according to Example 3 are sprayed on, first of all for 2 h at a spraying rate of 20 kg/h, then 24 kg/h and a spray pressure of 0.8 bar. The suspension is stirred constantly. The temperature of the air supplied is at most 75°C. The amount of air supplied is about 1900 m³/h.

Then the pellets are dried in the horizontal pan (5 revolutions per minute) at an air inflow temperature of at least 30°C, at most 50°C and an air inflow amount of 500 m³/h over a period of about 1-2 hours.

325 kg of the pellets thus obtained are then loaded once more into a horizontal pan and heated to 43°C. 900 kg of the suspension prepared previously according to Example 3 are sprayed on, first of all for 2 h at a spraying rate of 20 kg/h, then 24 kg/h and a spray pressure of 0.8 bar. The suspension is stirred constantly. The temperature of the air supplied is at most 75°C. The amount of air supplied is about 1900 m³/h.

Then the pellets are dried in the horizontal pan (5 revolutions per minute) at an air inflow temperature of at least 30°C, at most 50°C and an air inflow amount of 500 m³/h over a period of about 1-2 hours.

The dried pellets are then passed through a vibrating screen with a mesh size of 1.6 mm and stored in containers with desiccants until needed for further processing.

The following capsules were prepared based on the pellets obtained according to the process described above:

| **Component** | **[mg] per capsule** | **[mg] per capsule** |
|---|---|---|
| Dabigatran etexilate methanesulfonate | 86.48⁽¹⁾ | 126.83⁽²⁾ |
| Acacia (gum arabicum) | 4.43 | 6.50 |
| Tartaric acid | 88.56 | 129.9 |
| Hydroxymethylpropylcellulose 2910 | 2.23 | 3.27 |
| Dimethylpolysiloxane 350 | 0.04 | 0.06 |
| Talc | 17.16 | 25.16 |
| Hydroxypropylcellulose | 17.30 | 25.37 |
| HPMC-capsule | 60⁽³⁾ | 70⁽⁴⁾ |
| Total | 276.2 | 387.1 |
| ⁽¹⁾ equals 75 mg free dabigatran etexilate | | |
| ⁽²⁾ equals 110 mg free dabigatran etexilate | | |
| ⁽³⁾ Weight of the capsule approx. 60 mg | | |
| ⁽⁴⁾ Weight of the capsule approx. 70 mg | | |

| **Component** | **[mg] per capsule** |
|---|---|
| Dabigatran etexilate methanesulfonate | 172.95⁽¹⁾ |
| Acacia (gum arabicum) | 8.86 |
| Tartaric acid | 177.14 |
| Hydroxymethylpropylcellulose 2910 | 4.46 |
| Dimethylpolysiloxane 350 | 0.08 |
| Talc | 34.41 |
| Hydroxypropylcellulose | 34.59 |
| HPMC-capsule | 90⁽³⁾ |
| Total | 522.4 |
| ⁽¹⁾ equals 150 mg free dabigatran etexilate | |
| ⁽³⁾ Weight of the capsule approx. 90 mg | |

In the following section the experiments supporting the surprsingly high absorption of the drug on charcoal are described.

The contents of 5, 10 and 20 capsules (150 mg/capsule) of dabigatran etexilate were suspended in 100 ml water (approx. stomach volume, pH 2.7, 2.5 and 2.4, respectively). Each suspension was divided in half. To one portion a freshly prepared activated charcoal suspension (125 mg/ml, Ultracarbon®, Merck) was added. Both suspensions (with and without charcoal) were filtered and dabigatran etexilate concentrations were assessed via HPLC.

Levels of 8.3, 15.6 and 29.6 mg/ml dabigatran etexilate were recovered by HPLC in the untreated suspensions in water. In the charcoal-treated suspensions, the dabigatran etexilate peak was no longer detectable, this indicated that >99.9 % of dabigatran etexilate was adsorbed by the activated charcoal for all three tested concentrations.

In the plasma experiments, dabigatran was added to a plasma pool at supra-therapeutic concentrations of 470 and 940 ng/ml. The sample was then split, and active charcoal was added to half at the manufacturer's specified concentration (125 mg/ml), or 1:11 dilution of this. Dabigatran plasma levels were measured by a validated LC-MS/MS method.

After adding dabigatran active substance to plasma, values of 394 ± 19.4 and 824 ± 39.3 ng/ml (mean ± SD) were obtained in untreated plasma. Addition of activated charcoal at both concentrations reduced levels of dabigatran to <1.01 ng/ml (i.e. close to or below lower limit of quantification of the assay).

## Claims

1. Charcoal for use in the treatment of an overdose of active substance dabigatran etexilate
of formula I 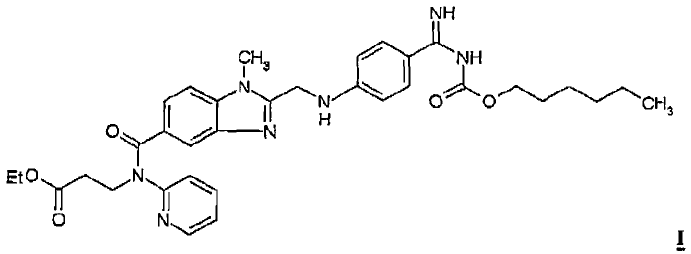 optionally in the form of the pharmaceutically acceptable salts thereof

2. Charcoal for the use according to claim 1, wherein the charcoal is administered orally.

3. Charcoal for the use according to claim 2, wherein the amount of orally administered charcoal is in a range of 20 - 130 g.

4. Charcoal for the neutralization of overdosed dabigatran etexilate of formula I 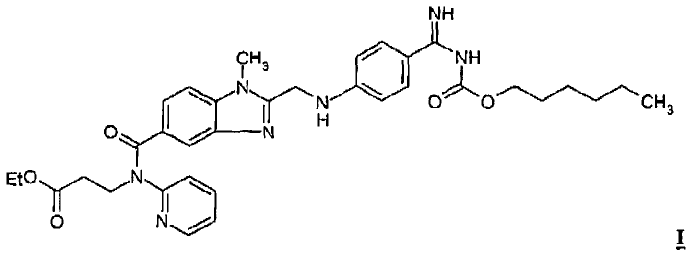 optionally in the form of the pharmaceutically acceptable salts thereof.

5. Charcoal for the use according to claim 4, wherein the charcoal is administered orally.

6. Charcoal for the use according to claim 5, wherein the amount of orally administered charcoal is in a range of 20 - 130 g.

7. Charcoal for the use of absorbing dabigatran **II** 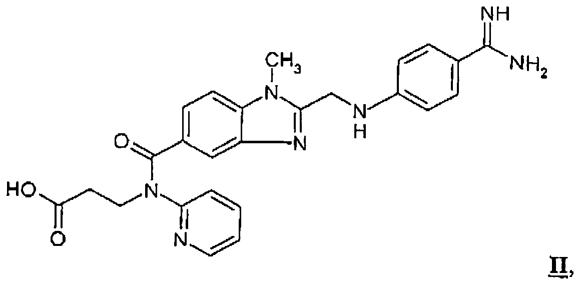 from blood plasma of a patient comprising the step of purifying the blood plasma over active charcoal.

8. Charcoal for the use according to claim 7, wherein the absorption of dabigatran **II** from the blood plasma is performed via hemoperfusion over a charcoal filter.

## Patentansprüche

1. Aktivkohle zur Verwendung in der Behandlung einer Überdosis der Wirksubstanz Dabigatranetexilat der Formel I 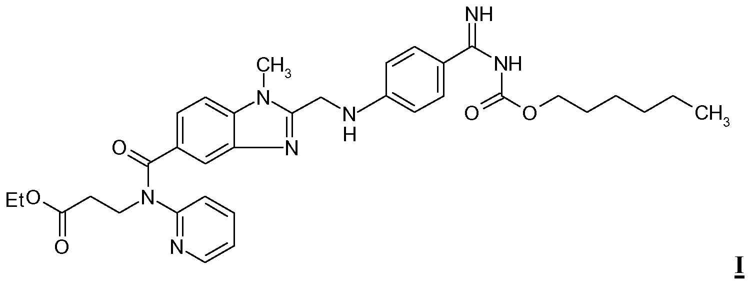 gegebenenfalls in Form der pharmazeutisch akzeptablen Salze davon.

2. Aktivkohle zur Verwendung nach Anspruch 1, wobei die Aktivkohle oral verabreicht wird.

3. Aktivkohle zur Verwendung nach Anspruch 2, wobei die Menge der oral verabreichten Aktivkohle in einem Bereich von 20 - 130 g liegt.

4. Aktivkohle zur Neutralisation von überdosiertem Dabigatranetexilat der Formel I 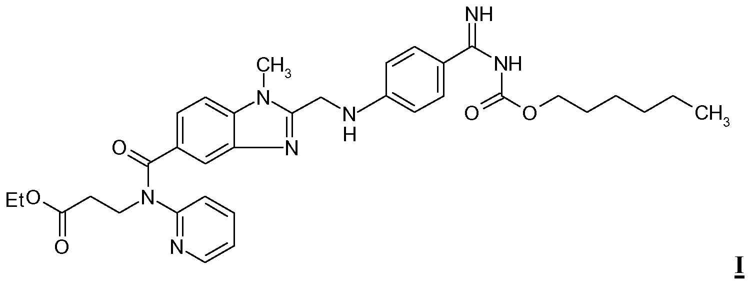 gegebenenfalls in Form der pharmazeutisch akzeptablen Salze davon.

5. Aktivkohle zur Verwendung nach Anspruch 4, wobei die Aktivkohle oral verabreicht wird.

6. Aktivkohle zur Verwendung nach Anspruch 5, wobei die Menge der oral verabreichten Aktivkohle in einem Bereich von 20 - 130 g liegt.

7. Aktivkohle zur Verwendung zum Absorbieren von Dabigatran **II** 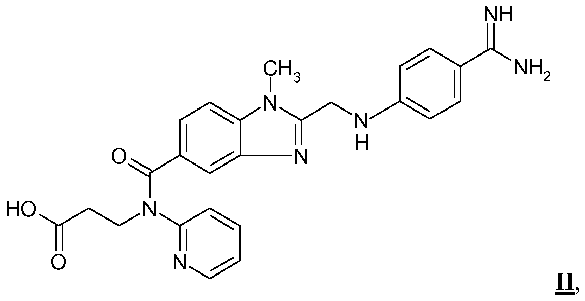 aus Blutplasma eines Patienten, umfassend den Schritt des Reinigens des Blutplasmas über Aktivkohle.

8. Aktivkohle zur Verwendung nach Anspruch 7, wobei die Absorption von Dabigatran **II** aus dem Blutplasma durch Hämoperfusion über einen Aktivkohlefilter durchgeführt wird.

## Revendications

1. Charbon pour une utilisation dans le traitement d'une surdose de substance active dabigatran étexilate de formule I 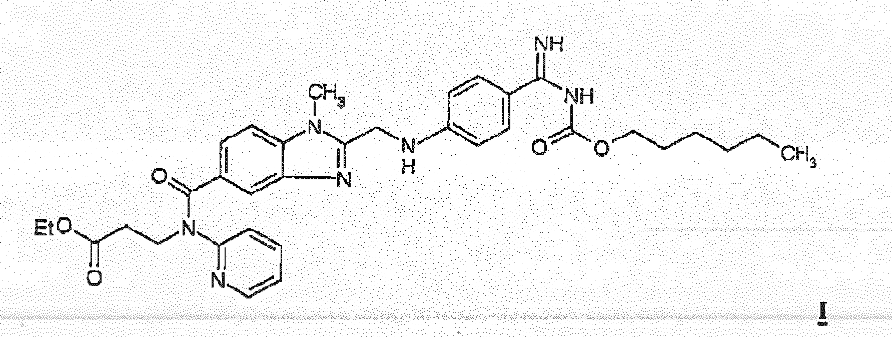 éventuellement sous la forme de ses sels pharmaceutiquement acceptables.

2. Charbon pour l'utilisation selon la revendication 1, dans lequel le charbon est administré oralement.

3. Charbon pour l'utilisation selon la revendication 2, dans lequel la quantité de charbon administré oralement est dans une plage de 20 à 130 g.

4. Charbon pour la neutralisation du dabigatran
étexilate surdosé de formule I 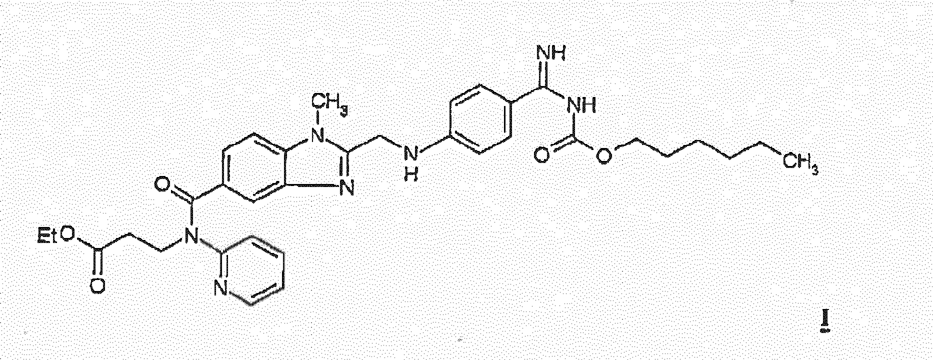 éventuellement sous la forme de ses sels pharmaceutiquement acceptables.

5. Charbon pour l'utilisation selon la revendication 4, dans lequel le charbon est administré oralement.

6. Charbon pour l'utilisation selon la revendication 5, dans lequel la quantité de charbon administré oralement est dans la plage de 20 à 130 g.

7. Charbon pour l'utilisation d'absorption du dabigatran II 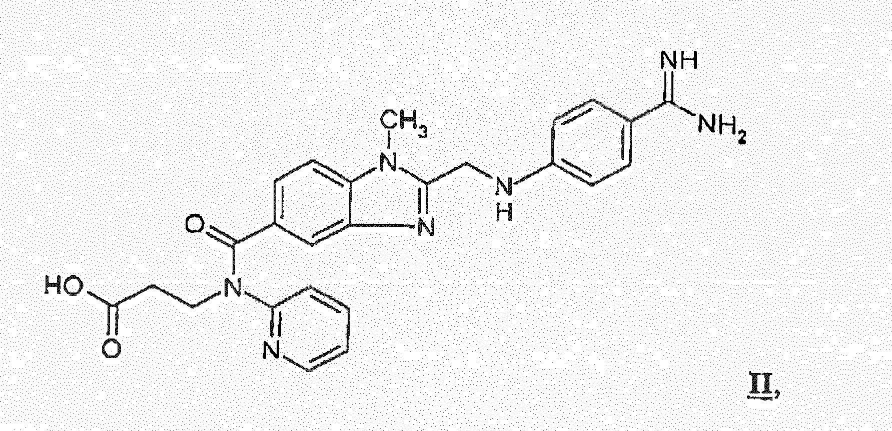 à partir du plasma sanguin d'un patient comprenant l'étape de purification du plasma sanguin sur du charbon activé.

8. Charbon pour l'utilisation selon la revendication 7, dans lequel l'absorption de dabigatran II à partir du plasma sanguin est effectuée via une hémoperfusion sur un filtre à charbon.
